# EUROPEAN PATENT APPLICATION

(11) **EP 3 796 329 A1**
(43) Date of publication of application: **24.03.2021**
(21) Application number: 20162337.8
(22) Date of filing: 11.03.2020
(51) Int. Cl.: G16H 40/00, A61M 5/315

(54) **INFORMATION COLLECTION DEVICE OF INJECTION PEN AND INFORMATION COLLECTION METHOD THEREOF**

(30) Priority: 18.09.2019 TW 108133664
(71) Applicant: Quanta Computer Inc., Taoyuan City 333 (TW)
(72) Inventor: CHENG, Kai-Ju, 333 Taoyuan City (TW); WU, Huan-Tang, 333 Taoyuan City (TW); SHEN, Huan-Pin, 333 Taoyuan City (TW)
(74) Representative: Winter, Brandl, Fürniss, Hübner, Röss, Kaiser, Polte - Partnerschaft mbB

(57) **Abstract**

An information collection method for an injection pen includes several steps as follows. The action of the pressing knob is sensed to determine whether the rotation information of the pressing knob is obtained. The traveling movement of the injection pen is sensed to determine whether the movement information of the injection pen is obtained. A determination is made that the traveling movement of the injection pen is conformed as an intending injection action according to the rotation information of the pressing knob and the movement information of the injection pen. When the traveling movement of the injection pen is conformed as the intending injection action, an actual injection dose that the injection content is pushed outwards from the injection pen is calculated according to the rotation information of the pressing knob. The actual injection dose is recorded.

## Description

### BACKGROUND

### Field of Disclosure

The disclosure relates to an information collection device and its information collection method. More particularly, the disclosure relates to an information collection device of an injection pen and its information collection method.

### Description of Related Art

In general, since diabetic patients need to inject insulin for the treatment of diabetes once or several times per day, most of them prefers to use a disposable insulin pen which is pre-filled therein so as to inject insulin to the body on their own.

However, an intelligent management system for insulin therapy provided by the current industry is unable to read the injection dose of the insulin injection pen injected on the diabetic patient, and unable to confirm whether the insulin injection pen has completed the intending injection action. Thus, the objective of the intelligent management system for diabetic patients is still not humanized, and the intelligent management system may cause many inconveniences and problems, thereby reducing the user's willingness to use.

### SUMMARY

In one embodiment of the disclosure, an information collection method for an injection pen, which is implemented on an information collection device coupled to a pressing knob of the injection pen, and the pressing knob is able to be pressed to helically rotate on the injection pen so that an injection content is pushed outwards from the injection pen, and the information collection method includes several steps as follows. The action of the pressing knob is sensed to determine whether the rotation information of the pressing knob is obtained. The traveling movement of the injection pen is sensed to determine whether the movement information of the injection pen is obtained. A determination is made as to whether the traveling movement of the injection pen is conformed as an intending injection action according to the rotation information of the pressing knob and the movement information of the injection pen. When the traveling movement of the injection pen is conformed as the intending injection action, an actual injection dose that the injection content is pushed outwards from the injection pen is calculated according to the rotation information of the pressing knob. The actual injection dose that the injection content is pushed outwards from the injection pen is recorded.

According to one or more embodiments of the disclosure, in the information collection method for the injection pen, when the traveling movement of the injection pen not conformed as the intending injection action is determined, the rotation information of the pressing knob is set to be invalid data, and the actual injection dose is not recorded.

According to one or more embodiments of the disclosure, in the information collection method for the injection pen, when a determination is made as to whether the traveling movement of the injection pen being conformed as the intending injection action, the information collection method further includes several steps as follows. A determination is made as to whether the acceleration information of the injection pen is in a predetermined range. When the acceleration information of the injection pen not in the predetermined range is determined, the traveling movement of the injection pen not conformed to be the intending injection action is decided.

According to one or more embodiments of the disclosure, in the information collection method for the injection pen, when a determination is made as to whether the traveling movement of the injection pen being conformed as the intending injection action, the information collection method further includes several steps as follows. A determination is made as to whether the injection pen is travelled in a moving direction along a shaft axis of the injection pen. When the injection pen not travelled in the moving direction along the shaft axis of the injection pen is determined, the traveling movement of the injection pen not conformed to be the intending injection action is decided.

According to one or more embodiments of the disclosure, in the information collection method for the injection pen, when a determination is made as to whether the traveling movement of the injection pen is conformed as the intending injection action according to the rotation information of the pressing knob and the movement information of the injection pen, the information collection method further includes several steps as follows. A determination is made as to whether a rotational angular velocity of the pressing knob is obtained. When the rotational angular velocity of the pressing knob been obtained is determined, a determination is made as to whether the injection pen is travelled in a moving direction along a Z-axis direction of a coordinate system. When the injection pen travelled in a moving direction along the Z-axis direction of the coordinate system, a determination is made as to whether a magnitude of the injection pen that is travelled in the moving direction along one of an X-axis direction and a Y-axis direction of the coordinate system is zero. When the magnitude of the injection pen that is travelled in the moving direction along one of the X-axis direction and the Y-axis direction of the coordinate system is zero is determined, the traveling movement of the injection pen being conformed as the intending injection action is decided.

In another embodiment of the disclosure, an information collection device for an injection pen includes an outer cap, an accelerometer sensing unit, a gyroscope sensing unit, a wireless transmission unit and a processing unit. The outer cap is used for coupling to a pressing knob of the injection pen, and the pressing knob is able to be pressed to helically rotate on the injection pen so that the injection content is pushed outwards from the injection pen. The accelerometer sensing unit is disposed on the outer cap for sensing a traveling movement of the injection pen to obtain the movement information of the injection pen. The gyroscope sensing unit is disposed on the outer cap for sensing an action of the pressing knob to obtain the rotation information of the pressing knob. The wireless transmission unit is electrically connected to the gyroscope sensing unit and the accelerometer sensing unit for wirelessly connecting to an external device. The processing unit is electrically connected to the wireless transmission unit, the gyroscope sensing unit and the accelerometer sensing unit. When the pressing knob is pressed to helically rotate, the processing unit determines whether the traveling movement of the injection pen is conformed as an intending injection action according to the rotation information of the pressing knob and the movement information of the injection pen. When the processing unit determines that the traveling movement of the injection pen is conformed as the intending injection action, the processing unit instructs the wireless transmission unit to transmit one of the rotation information of the pressing knob and an actual injection dose of the injection content being pushed outwards from the injection pen to the external device.

According to one or more embodiments of the disclosure, the information collection device further includes a circuit board and a trigger switch. The circuit board is fixed in the outer cap, and the processing unit, the wireless transmission unit, the gyroscope sensing unit and the accelerometer sensing unit are respectively fixed on the circuit board. The trigger switch is fixedly connected to the circuit board, and electrically connected to the processing unit, the gyroscope sensing unit, the accelerometer sensing unit and the wireless transmission unit for triggering the processing unit, the gyroscope sensing unit, the accelerometer sensing unit and the wireless transmission unit.

According to one or more embodiments of the disclosure, in the information collection device, the outer cap further includes a cover body, a joint portion, an opening and a button body. The cover body is formed with a receiving recess at one end of the cover body, and the pressing knob, the processing unit, the wireless transmission unit, the gyroscope sensing unit and the accelerometer sensing unit are received within the receiving recess. The joint portion is formed at the end of the cover body for removably coupling to the pressing knob of the injection pen. The opening is formed at one end being opposite to the cover body, and the receiving recess is connected to the joint portion and the opening. The button body is movably disposed in the receiving recess, and one end of the button body extends outwards from the end of the cover body via the opening, and the other end of the button body abuts against the trigger switch in the receiving recess.

According to one or more embodiments of the disclosure, in the information collection device, the processing unit determines whether the acceleration information of the injection pen is in a predetermined range. When the processing unit determines that the acceleration information of the injection pen is not in the predetermined range, the processing unit decides that the traveling movement of the injection pen is not conformed to be the intending injection action.

According to one or more embodiments of the disclosure, in the information collection device, the processing unit determines whether the injection pen is travelled in a moving direction along a shaft axis of the injection pen. When the processing unit determines that the injection pen is not travelled in the moving direction along the shaft axis of the injection pen, the processing unit decides that the traveling movement of the injection pen is not conformed to be the intending injection action.

Thus, through the construction of the embodiments above, not only the actual injection dose of the injection pen can be accurately recorded, but also a confirmation can be made as to whether the injection pen has completed the intending injection action, thereby avoiding recording false values from non-injection behavior. Therefore, the user-friendly design is improved, and the inconvenience and troubles described above are solved, thereby increasing the user's willingness to use.

The above description is merely used for illustrating the problems to be resolved, the technical methods for resolving the problems and their efficacies, etc. The specific details of the disclosure will be explained in the embodiments below and related drawings.

### BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure. In the drawings,
Fig. 1 is an exploding view of an injection module according to one embodiment of the disclosure;
Fig. 2 is a block diagram of the information collection device of Fig. 1;
Fig. 3 is a partial cross-sectional view of the injection module of Fig. 1;
Fig. 4 is an operation flow chart of the injection module of Fig. 1; and
Fig. 5 is a flow chart of an information collection method for an injection pen according to one embodiment of the disclosure.

### DESCRIPTION OF THE EMBODIMENTS

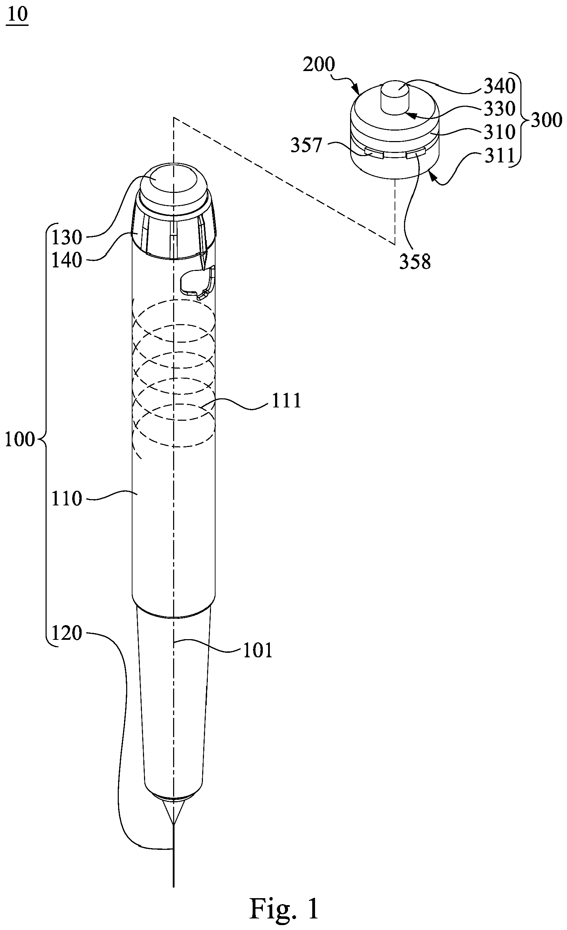

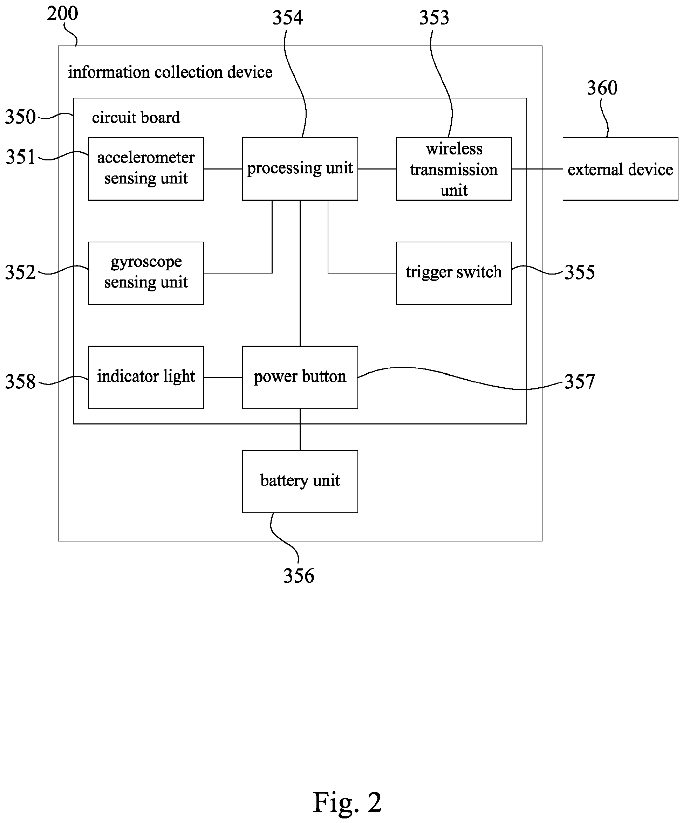

Reference is now made to Fig. 1 and Fig. 2, in which Fig. 1 is an exploding view of an injection module 10 according to one embodiment of the disclosure, and Fig. 2 is a block diagram of the information collection device 200 of Fig. 1. As shown in Fig. 1 and Fig. 2, the information collection device 200 is used to be sleeved on an injection pen 100 to be collectively referred as an injection module 10. The injection pen 100 includes a syringe 110, an injection needle 120, a pressing knob 130 and a dose selection knob 140. The syringe 110 is formed with a spiral inner rail 111 therein. The injection needle 120 and the pressing knob 130 are disposed at two opposite ends of the syringe 110, respectively. The pressing knob 130 can be pressed to helically rotate so that an injection content (not shown) that is filled in the syringe 110 can be simultaneously pushed outwards from the syringe 110 through the injection needle 120. The dose selection knob 140 is located at one end of the syringe 110 that is closer to the pressing knob 130 for determining the expected injection dose by rotation.

In this embodiment, for example, the injection pen 100 is an insulin injection pen. When the pressing knob 130 is rotated by a user, that is, the user presses the pressing knob 130 downwardly, so that the pressing knob 130 moves along the spiral inner rail 111 with helical movement so as to gradually push the injection content (not shown) outwards from the syringe 110 through the injection needle 120. The pressing knob is able to be helically rotated about a shaft axis 101 of the injection pen 100. Since the injection pen is a conventional device, it will not be described again hereinafter. However, the disclosure is not limited to use on an insulin injection pen only.

When the intending injection action is completed, the information collection device 200 is used to record an estimated injected dose of the injection pen 100 that is supposed to be the actual injected dose of the injection pen 100. In short, the information collection device 200 includes an outer cap 300, an accelerometer sensing unit 351, a gyroscope sensing unit 352, a wireless transmission unit 353 and a processing unit 354. The outer cap 300 is used for coupling to the pressing knob 130 of the injection pen 100. The accelerometer sensing unit 351 is disposed on the outer cap 300 for sensing a traveling movement of the injection pen 100 with the movement information of the injection pen 100. The movement information of the injection pen 100 is, for example, the moving direction and acceleration information of the injection pen 100, and the like. The gyroscope sensing unit 352 is disposed on the outer cap 300 for sensing an action of the pressing knob 130 to obtain the rotation information of the pressing knob 130. The rotation information of the pressing knob 130 is, for example, a rotational angular velocity, and the rotation information can be converted into a rotation starting point and a rotation endpoint of a rotation variable. It is noted, different actual injection doses of the injection content (not shown) can be respectively corresponded to different rotation variables of the pressing knob 130 that is rotated. The wireless transmission unit 353 is electrically connected to the gyroscope sensing unit 352 and the accelerometer sensing unit 351 for wirelessly connecting to an external device 360. The wireless transmission unit 353 is, for example, a radio frequency (RF) transmitting unit. The processing unit 354 is electrically connected to the wireless transmission unit 353, the gyroscope sensing unit 352 and the accelerometer sensing unit 351. Thus, when the aforementioned pressing knob 130 is pressed down to helically rotate by a user, according to the rotation information of the pressing knob and the movement information of the injection pen, the processing unit 354 determines whether the traveling movement of the injection pen is conformed as an intending injection action, or other non-intending injection action such as accidental touch, movement and alike. When the processing unit 354 decides that the traveling movement of the injection pen is conformed as the intending injection action, the processing unit 354 instructs the wireless transmission unit 353 to transfer an actual injected dose of the injection content that is injected from the injection pen 100 to the external device 360 for recording the actual injected dose to the external device 360. More specifically, the processing unit 354 calculates an actual injection dose of the injection content being pushed outwards from the injection pen 100 according to the rotation information (e.g., rotation amount) of the pressing knob 130. The processing unit 354 instructs the radio frequency (RF) transmitting unit to transfer the actual injected dose of the injection content to the external device 360. The external device 360 is, for example, a portable device (e.g., smart mobile phone or notebook computer) or a cloud device (e.g., server) or the like. However, the disclosure is not limited thereto. In other embodiments, the processing unit 354 may not process the rotation information into the actual injection dose first, and transfer the rotation information to the external device 360 through the wireless transmission unit 353 so that the external device 360 can process the rotation information into the actual injection dose by itself.

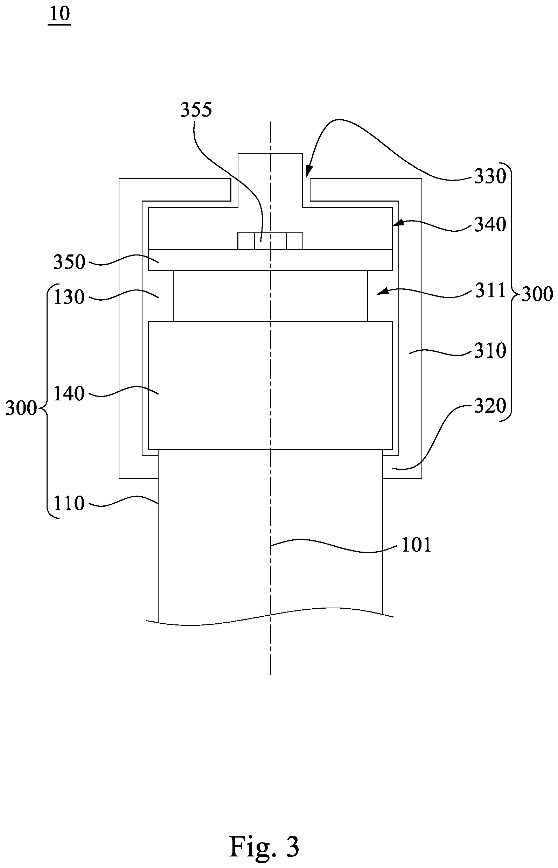

Fig. 3 is a partial cross-sectional view of the injection module 10 of Fig. 1. As shown in Fig. 2 and Fig. 3, in the embodiment, the information collection device 200 further includes a circuit board 350 and a trigger switch 355. The circuit board 350 is fixed in the outer cap 300, and the processing unit 354, the wireless transmission unit 353, the gyroscope sensing unit 352 and the accelerometer sensing unit 351 are respectively fixed on the circuit board 350. For example, the processing unit 354, the wireless transmission unit 353, the gyroscope sensing unit 352 and the accelerometer sensing unit 351 are respectively soldered on the circuit board 350, and are electrically connected to each other. The trigger switch 355 is fixedly connected to the circuit board 350, and electrically connected to the processing unit 354, the gyroscope sensing unit 352, the accelerometer sensing unit 351 and the wireless transmission unit 353 for triggering the processing unit 354, the gyroscope sensing unit 352, the accelerometer sensing unit 351 and the wireless transmission unit 353.

Furthermore, more specifically, the outer cap 300 further includes a cover body 310, a joint portion 320, an opening 330 and a button body 340. The cover body 310 is formed with a receiving recess 311 at one end of the cover body 310, and the pressing knob 130, the processing unit 354, the wireless transmission unit 353, the gyroscope sensing unit 352 and the accelerometer sensing unit 351 are received within the receiving recess 311. The joint portion 320 is formed at the end of the cover body 310 for removably coupling to the pressing knob 130 of the injection pen 100. The opening 330 is formed at one end being opposite to the cover body 310, and the receiving recess 311 is connected to the joint portion 320 and the opening 330. The button body 340 is movably disposed in the receiving recess 311, and one end of the button body 340 extends outwards from the end of the cover body 310 via the opening 330, and the other end of the button body 340 abuts against the trigger switch 355 in the receiving recess 311.

Moreover, the information collection device 200 further includes a battery unit 356, a power button 357 and an indicator light 358. The battery unit 356 is electrically connected to the processing unit 354, the wireless transmission unit 353, the gyroscope sensing unit 352, the accelerometer sensing unit 351, the power button 357 and the indicator light 358 through the circuit board 350 to respectively provide corresponding power supplies. Indicator light 358 is used to indicate the operating status.

Also, in the embodiment, the accelerometer sensing unit 351 and the gyroscope sensing unit 352 may be integrally combined into a single package structure, for example, a six-axis sensor, however, the disclosure is not limited thereto.

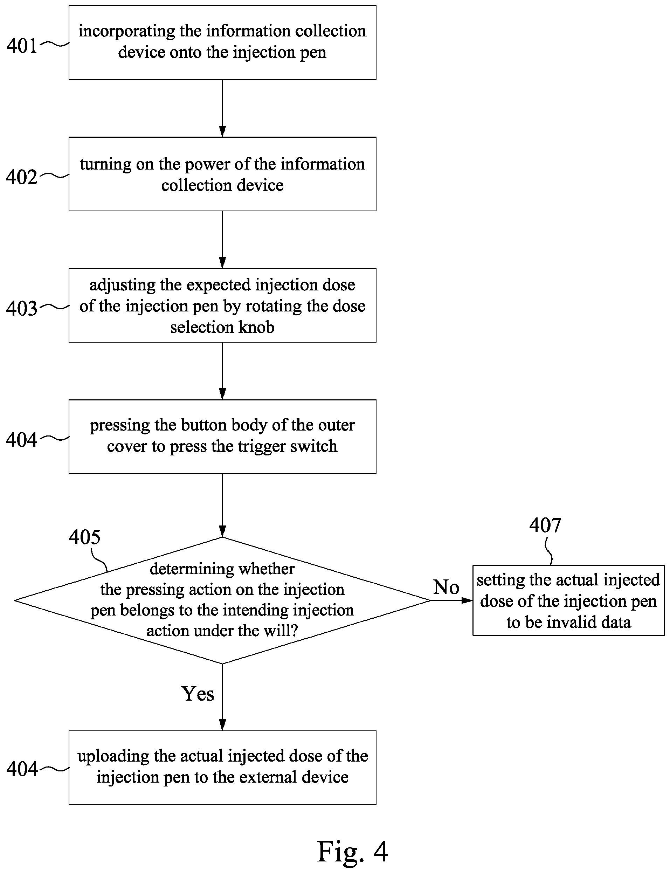

Fig. 4 is an operation flow chart of the injection module 10 of Fig. 1. As shown in Fig. 2 and Fig. 3, in the embodiment, the user uses the injection module 10 according to the following step 401 to step 407. In step 401, a user incorporates the information collection device 200 onto the injection pen 100. In step 402, the user turns on the power of the information collection device 200. More specifically, the power button 357 is pressed so that the information collection device 200 starts to stand by to wait for subsequent triggering. In step 403, the user adjusts the expected injection dose of the injection pen 100 by rotating the dose selection knob 140. In step 404, the user presses the button body 340 of the outer cap 300, thereby pressing the trigger switch 355. More specifically, when the user wants to inject the above-described injection pen 100, the user presses the trigger switch 355 through the button body 340 and presses the knob 130. In step 405, when the pressing knob 130 is rotated by the user, the processing unit 354 starts to determine whether the user's pressing action on the injection pen 100 belongs to the intending injection action under the will. If yes, go to step 406; otherwise, perform the step 407. In step 406, the actual injected dose of the injection pen 100 that is an estimated injected dose, is transmitted (or uploaded) to the external device 360 for recording the actual injected dose.

In step 407, the actual injected dose of the injection pen 100 is set to be invalid data, and is not passed to the external device 360 or not recorded.

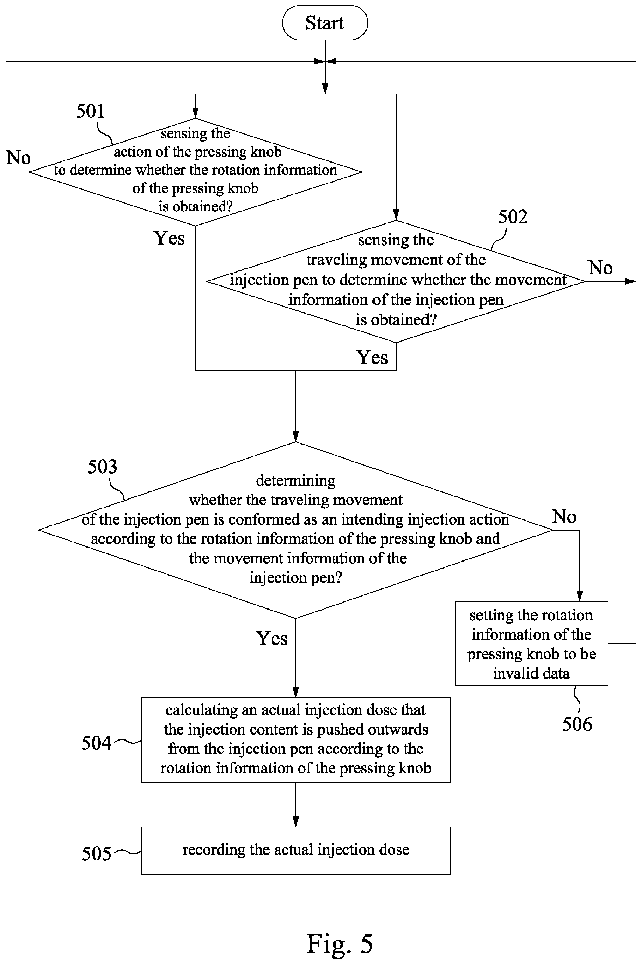

Fig. 5 is a flow chart of an information collection method for an injection pen 100 according to one embodiment of the disclosure. As shown in Fig. 5, in the embodiment, the information collection method (i.e., step 405) for the injection pen 100 includes step 501 to step 506 as follows. In step 501, the action of the pressing knob 130 is sensed to determine whether the rotation information of the pressing knob 130 is obtained. In step 502, the traveling movement of the injection pen 100 is sensed to determine whether the movement information of the injection pen 100 is obtained. In step 503, a determination is made as to whether the traveling movement of the injection pen 100 is conformed as an intending injection action according to the rotation information of the pressing knob and the movement information of the injection pen 100; if, yes, perform step 504, otherwise, perform step 506. In step 504, the actual injection dose that the injection content is pushed outwards from the injection pen 100 is calculated according to the rotation information of the pressing knob 130. In step 505, the actual injection dose that the injection content is pushed outwards from the injection pen 100 is recorded. In step 506, the rotation information of the pressing knob 130 is set to be invalid data. For example, step 501 to step 506 are the detailed steps of the abovementioned step 405.

As shown in Fig. 2 and 5, in the disclosure, when the pressing knob 130 is triggered, the processing unit 354 starts to perform the above-described step 501 to step 506.

More specifically, in step 501, the rotation information of the pressing knob 130 is sensed by the gyroscope sensing unit 352. For example, the rotation information of the pressing knob 130 is a rotational angular velocity, and the rotation information can be converted into a rotation starting point and a rotation endpoint of a rotation variable. Different actual injection doses of the injection content (not shown) can be respectively corresponded to different rotation variables of the pressing knob 130 that is rotated.

In this embodiment, more specifically, step 502 further includes a step of the movement information of the injection pen 100 is sensed by the accelerometer sensing unit 351. The movement information of the injection pen 100 is, for example, the moving direction and acceleration information of the injection pen 100, and the like.

In this embodiment, more specifically, the step 503 further includes the current rotation variable that is obtained through a rotation starting point and a rotation endpoint of the rotation information; and a determination is made as to whether the current rotation variable is within a predetermined range, and/or the rotational angular velocity is within a predetermined range. If so, it is determined that the rotation variable and the rotational angular velocity of the pressing knob 130 are valid data, otherwise, perform step 506.

In this embodiment, more specifically, when the rotation variable and the rotational angular velocity of the pressing knob 130 are determined as the valid data, step 503 further includes a determination is made as to whether the travelling information is greater than a predetermined range through the sensing information of the accelerometer sensing unit 351. If so, the traveling movement of the injection pen 100 not conformed as the intending injection action is decided, for example, the traveling movement of the injection pen100 merely is shaking or accidentally touching the trigger switch 355. Otherwise, the traveling movement of the injection pen 100 conformed as the intending injection action is decided, for example, an injection behavior.

Thus, since the rotation variable of the pressing knob 130 is valid data, and the traveling movement of the injection pen 100 is decided to be the intending injection action, thus, step 504 can be performed.

However, the disclosure is not limited thereto, in other embodiment, in step 505, more particularly, a determination is made as to whether the injection pen 100 is travelled in a moving direction along a shaft axis 101 of the injection pen 100 through the moving direction of the travelling information of the injection pen 100. If so, the traveling movement of the injection pen 100 conformed as the intending injection action is decided, for example, the traveling movement of the injection pen 100 is an injection behavior. Otherwise, the traveling movement of the injection pen 100, for example, merely shakes or accidentally touches the trigger switch 355.

In more detail, when the shaft axis 101 of the injection pen 100 is considered as one of the X-axis direction, the Y-axis direction and the Z-axis direction of the coordinate system (e.g., Z-axis direction), a determination is made as to whether a movement amount that the injection pen 100 is moved along the shaft axis 101 of the injection pen 100 is within a predetermined range; if so, a determination is made as to whether the movement amount that the injection pen 100 is moved along the shaft axis 101 of the injection pen 100 is greater than another two of the X-axis direction, the Y-axis direction and the Z-axis direction of the coordinate system (e.g., X and Y-axis directions); if so, the current traveling movement of the injection pen 100 is decided as a purposely action. In addition, when the rotational angular velocity collected from the rotation of the injection pen 100 is decided, a determination is further made as to whether the moving direction of the injection pen 100 is moved along the Z-axis direction of a coordinate system (for example, Cartesian coordinate system), if so, a determination is further made as to whether a magnitude of the injection pen 100 that is travelled in the moving direction along an X-axis direction or a Y-axis direction of the coordinate system is zero, the current traveling movement of the injection pen 100 is decided as a purposely action, for example, injection action.

Furthermore, more specifically, after step 504, the method mentioned above further includes several steps as follows. The rotational angular velocity collected by pressing the pressing knob 130 is converted into an expected injection dose of the injection content; and the expected injection dose of the injection content is wirelessly transmitted to the external device 360 for checking the consistency of the actual injected dose and the expected injected dose.

It is noted, the step 504 mentioned above may be completed in the information collection device 200 or may be completed by the external device 360.

Thus, through the construction of the embodiments above, not only the actual injection dose of the injection pen can be accurately recorded, but also a confirmation can be made as to whether the injection pen has completed the intending injection action, thereby avoiding recording false values from non-injection behavior. Therefore, the user-friendly design is improved, and the inconvenience and troubles described above are solved, thereby increasing the user's willingness to use.

It will be apparent to those skilled in the art that various modifications and variations can be made to the structure of the disclosure without departing from the scope or spirit of the disclosure. In view of the foregoing, it is intended that the disclosure cover modifications and variations of this disclosure provided they fall within the scope of the following claims and their equivalents.

## Claims

1. An information collection method for an injection pen (100), which is implemented on an information collection device (300) coupled to a pressing knob (130) of the injection pen (100), and the pressing knob (130) is able to be pressed to helically rotate on the injection pen (100) so that an injection content is pushed outwards from the injection pen (100), and the information collection method **characterized by** comprising:
sensing an action of the pressing knob (130) to determine whether a rotation information of the pressing knob (130) is obtained;
sensing a traveling movement of the injection pen (100) to determine whether a movement information of the injection pen (100) is obtained;
determining whether the traveling movement of the injection pen (100) is conformed as an intending injection action according to the rotation information of the pressing knob (130) and the movement information of the injection pen (100); and
when determining that the traveling movement of the injection pen (100) is conformed as the intending injection action, calculating an actual injection dose that the injection content is pushed outwards from the injection pen (100) according to the rotation information of the pressing knob (130); and
recording the actual injection dose that the injection content is pushed outwards from the injection pen (100).

2. The information collection method for the injection pen (100) of claim 1, **characterized in that** when determining that the traveling movement of the injection pen (100) is not conformed as the intending injection action, setting the rotation information of the pressing knob (130) to be invalid data, and the actual injection dose is not recorded.

3. The information collection method for the injection pen (100) of claim 2, **characterized in that** determining whether the traveling movement of the injection pen (100) is conformed as the intending injection action according to the rotation information of the pressing knob (130) and the movement information of the injection pen (100), further comprises:
determining whether an acceleration information of the injection pen (100) is in a predetermined range; and
when determining that the acceleration information of the injection pen (100) is not in the predetermined range, deciding the traveling movement of the injection pen (100) is not conformed to be the intending injection action.

4. The information collection method for the injection pen (100) of claim 2, **characterized in that** determining whether the traveling movement of the injection pen (100) is conformed as the intending injection action according to the rotation information of the pressing knob (130) and the movement information of the injection pen (100), further comprises:
determining whether the injection pen (100) is travelled in a moving direction along a shaft axis (101) of the injection pen (100); and
when determining that the injection pen (100) is not travelled in the moving direction along the shaft axis (101) of the injection pen (100), deciding the traveling movement of the injection pen (100) is not conformed to be the intending injection action.

5. The information collection method for the injection pen (100) of claim 1, **characterized in that** determining whether the traveling movement of the injection pen (100) is conformed as the intending injection action according to the rotation information of the pressing knob (130) and the movement information of the injection pen (100), further comprises:
determining whether a rotational angular velocity of the pressing knob (130) is obtained;
when determining that the rotational angular velocity of the pressing knob (130) is obtained, determining whether the injection pen (100) is travelled in a moving direction along a Z-axis direction of a coordinate system;
when determining that the injection pen (100) is travelled in the moving direction along the Z-axis direction of the coordinate system, determining whether a magnitude of the injection pen (100) that is travelled in the moving direction along one of an X-axis direction and a Y-axis direction of the coordinate system is zero; and
when determining that the magnitude of the injection pen (100) being travelled in the moving direction along one of the X-axis direction and the Y-axis direction of the coordinate system is zero, deciding the traveling movement of the injection pen (100) is conformed as the intending injection action.

6. An information collection device (300) for an injection pen (100), **characterized by** comprising:
an outer cap (300) for coupling to a pressing knob (130) of the injection pen (100), wherein the pressing knob (130) is able to be pressed to helically rotate on the injection pen (100) so that an injection content is pushed outwards from the injection pen (100);
an accelerometer sensing unit (351) that is disposed on the outer cap (300) for sensing a traveling movement of the injection pen (100) to obtain a movement information of the injection pen (100);
a gyroscope sensing unit (352) that is disposed on the outer cap (300) for sensing an action of the pressing knob (130) to obtain a rotation information of the pressing knob (130);
a wireless transmission unit (353) that is electrically connected to the gyroscope sensing unit (352) and the accelerometer sensing unit (351) for wirelessly connecting to an external device (360); and
a processing unit (354) that is electrically connected to the wireless transmission unit (353), the gyroscope sensing unit (352) and the accelerometer sensing unit (351),
wherein when the pressing knob (130) is pressed to helically rotate, the processing unit (354) determines whether the traveling movement of the injection pen (100) is conformed as an intending injection action according to the rotation information of the pressing knob (130) and the movement information of the injection pen (100); and
when the processing unit (354) determines that the traveling movement of the injection pen (100) is conformed as the intending injection action, the processing unit (354) instructs the wireless transmission unit (353) to transmit one of the rotation information of the pressing knob (130) and an actual injection dose of the injection content being pushed outwards from the injection pen (100) to the external device (360).

7. The information collection device for the injection pen (100) of claim 6, further **characterized by** comprising:
a circuit board (350) that is fixed in the outer cap (300), wherein the processing unit (354), the wireless transmission unit (353), the gyroscope sensing unit (352) and the accelerometer sensing unit (351) are respectively fixed on the circuit board (350); and
a trigger switch (355) that is fixedly connected to the circuit board (350), and electrically connected to the processing unit (354), the gyroscope sensing unit (352), the accelerometer sensing unit (351) and the wireless transmission unit (353) for triggering the processing unit (354), the gyroscope sensing unit (352), the accelerometer sensing unit (351) and the wireless transmission unit (353).

8. The information collection device (300)for the injection pen (100) of claim 7, **characterized in that** the outer cap (300) comprising:
a cover body (310) that is formed with a receiving recess (311) at one end of the cover body (310), wherein the pressing knob (130), the processing unit (354), the wireless transmission unit (353), the gyroscope sensing unit (352) and the accelerometer sensing unit (351) are received within the receiving recess (311);
a joint portion (320) that is formed at the end of the cover body (310) for removably coupling to the pressing knob (130) of the injection pen (100);
an opening (330) that is formed at one end being opposite to the cover body (310), wherein the receiving recess (311) is connected to the joint portion (320) and the opening (310); and
a button body (340) that is movably disposed in the receiving recess (311), and one end of the button body (340) extends outwards from the end of the cover body (310) via the opening (310), and the other end of the button body (340) abuts against the trigger switch (355) in the receiving recess (311).

9. The information collection device for the injection pen (100) of claim 6, **characterized in that** the processing unit (354) determines whether an acceleration information of the injection pen (100) is in a predetermined range, and
when the processing unit (354) determines that the acceleration information of the injection pen (100) is not in the predetermined range, the processing unit (354) decides that the traveling movement of the injection pen (100) is not conformed to be the intending injection action.

10. The information collection device(300) for the injection pen (100) of claim 6, **characterized in that** the processing unit (354) determines whether the injection pen (100) is travelled in a moving direction along a shaft axis (101) of the injection pen (100), and
when the processing unit (354) determines that the injection pen (100) is not travelled in the moving direction along the shaft axis (101) of the injection pen (100), the processing unit (354) decides that the traveling movement of the injection pen (100) is not conformed to be the intending injection action.

## Amended claims

### Amended claims in accordance with Rule 137(2) EPC.

1. An information collection method for an injection pen (100), which is implemented on an information collection device (300) coupled to a pressing knob (130) of the injection pen (100), and the pressing knob (130) is able to be pressed to helically rotate on the injection pen (100) so that an injection content is pushed outwards from the injection pen (100), and the information collection method **characterized by** comprising:
sensing an action of the pressing knob (130) whereby a rotation information of the pressing knob (130) is obtained;
sensing a traveling movement of the injection pen (100) whereby a movement information of the injection pen (100) is obtained;
determining whether the traveling movement of the injection pen (100) conforms to an intended injection action according to the rotation information of the pressing knob (130) and the movement information of the injection pen (100), wherein determining whether the traveling movement of the injection pen (100) conforms to the intended injection action further comprising: determining whether the injection pen (100) is travelled in an axial moving direction along a shaft axis (101) of the injection pen (100), and when a determination is made of that the injection pen (100) is travelled in the axial moving direction along the shaft axis (101) of the injection pen (100), deciding the traveling movement of the injection pen (100) conforms to the intended injection action; and
when determining that the traveling movement of the injection pen (100) conforms to the intended injection action, calculating an actual injection dose that the injection content is pushed outwards from the injection pen (100) according to the rotation information of the pressing knob (130); and
recording the actual injection dose by which the injection content is pushed outwards from the injection pen (100).

2. The information collection method for the injection pen (100) of claim 1, **characterized in that** when determining that the traveling movement of the injection pen (100) not conforms to the intended injection action, setting the rotation information of the pressing knob (130) to be invalid data, and the actual injection dose is not recorded.

3. The information collection method for the injection pen (100) of claim 2, **characterized in that** determining whether the traveling movement of the injection pen (100) conforms to the intended injection action according to the rotation information of the pressing knob (130) and the movement information of the injection pen (100), further comprises:
determining whether an acceleration information of the injection pen (100) is in a predetermined range; and
when determining that the acceleration information of the injection pen (100) is not in the predetermined range, deciding the traveling movement of the injection pen (100) not conforms to the intended injection action.

4. The information collection method for the injection pen (100) of claim 2, **characterized in that** determining whether the traveling movement of the injection pen (100) conforms to the intended injection action according to the rotation information of the pressing knob (130) and the movement information of the injection pen (100), further comprises:
when determining that the injection pen (100) is not travelled in the moving direction along the shaft axis (101) of the injection pen (100), deciding the traveling movement of the injection pen (100) not conforms to the intended injection action.

5. The information collection method for the injection pen (100) of claim 1, **characterized in that** determining whether the traveling movement of the injection pen (100) conforms to the intended injection action according to the rotation information of the pressing knob (130) and the movement information of the injection pen (100), further comprises:
determining whether a rotational angular velocity of the pressing knob (130) is obtained;
when determining that the rotational angular velocity of the pressing knob (130) is obtained, determining whether the injection pen (100) is travelled in a moving direction along a Z-axis direction of a coordinate system;
when determining that the injection pen (100) is travelled in the moving direction along the Z-axis direction of the coordinate system, determining whether a magnitude of the injection pen (100) that is travelled in the moving direction along one of an X-axis direction and a Y-axis direction of the coordinate system is zero; and
when determining that the magnitude of the injection pen (100) being travelled in the moving direction along one of the X-axis direction and the Y-axis direction of the coordinate system is zero, deciding the traveling movement of the injection pen (100) conforms to the intended injection action.

6. An information collection device (300) for an injection pen (100), **characterized by** comprising:
an outer cap (300) for coupling to a pressing knob (130) of the injection pen (100), wherein the pressing knob (130) is able to be pressed to helically rotate on the injection pen (100) so that an injection content is pushed outwards from the injection pen (100);
an accelerometer sensing unit (351) that is disposed on the outer cap (300) for sensing a traveling movement of the injection pen (100) to obtain a movement information of the injection pen (100);
a gyroscope sensing unit (352) that is disposed on the outer cap (300) for sensing an action of the pressing knob (130) to obtain a rotation information of the pressing knob (130);
a wireless transmission unit (353) that is electrically connected to the gyroscope sensing unit (352) and the accelerometer sensing unit (351) for wirelessly connecting to an external device (360); and
a processing unit (354) that is electrically connected to the wireless transmission unit (353), the gyroscope sensing unit (352) and the accelerometer sensing unit (351),
wherein when the pressing knob (130) is pressed to helically rotate, the processing unit (354) determines whether the traveling movement of the injection pen (100) conforms to an intended injection action according to the rotation information of the pressing knob (130) and the movement information of the injection pen (100), wherein the processing unit (354) determines that the injection pen (100) is travelled in an axial moving direction along a shaft axis (101) of the injection pen (100) according to the movement information of the injection pen (100), the processing unit (354) decides that the traveling movement of the injection pen (100) conforms to the intended injection action; and when the processing unit (354) determines that the traveling movement of the injection pen (100) conforms to the intended injection action, the processing unit (354) instructs the wireless transmission unit (353) to transmit one of the rotation information of the pressing knob (130) and an actual injection dose of the injection content being pushed outwards from the injection pen (100) to the external device (360).

7. The information collection device for the injection pen (100) of claim 6, further **characterized by** comprising:
a circuit board (350) that is fixed in the outer cap (300), wherein the processing unit (354), the wireless transmission unit (353), the gyroscope sensing unit (352) and the accelerometer sensing unit (351) are respectively fixed on the circuit board (350); and
a trigger switch (355) that is fixedly connected to the circuit board (350), and electrically connected to the processing unit (354), the gyroscope sensing unit (352), the accelerometer sensing unit (351) and the wireless transmission unit (353) for triggering the processing unit (354), the gyroscope sensing unit (352), the accelerometer sensing unit (351) and the wireless transmission unit (353).

8. The information collection device (300) for the injection pen (100) of claim 7, **characterized in that** the outer cap (300) comprising:
a cover body (310) that is formed with a receiving recess (311) at one end of the cover body (310), wherein the pressing knob (130), the processing unit (354), the wireless transmission unit (353), the gyroscope sensing unit (352) and the accelerometer sensing unit (351) are received within the receiving recess (311);
a joint portion (320) that is formed at the end of the cover body (310) for removably coupling to the pressing knob (130) of the injection pen (100);
an opening (330) that is formed at one end being opposite to the cover body (310), wherein the receiving recess (311) is connected to the joint portion (320) and the opening (310); and
a button body (340) that is movably disposed in the receiving recess (311), and one end of the button body (340) extends outwards from the end of the cover body (310) via the opening (310), and the other end of the button body (340) abuts against the trigger switch (355) in the receiving recess (311).

9. The information collection device for the injection pen (100) of claim 6, **characterized in that** the processing unit (354) determines whether an acceleration information of the injection pen (100) is in a predetermined range, and
when the processing unit (354) determines that the acceleration information of the injection pen (100) is not in the predetermined range, the processing unit (354) decides that the traveling movement of the injection pen (100) not conforms to the intended injection action.

10. The information collection device(300) for the injection pen (100) of claim 6, **characterized in that** when the processing unit (354) determines that the injection pen (100) is not travelled in the moving direction along the shaft axis (101) of the injection pen (100), the processing unit (354) decides that the traveling movement of the injection pen (100) not conforms to the intended injection action.
